Europäisches Patentamt

European Patent Office

Office européen des brevets

(11) Veröffentlichungsnummer: **0 449 865 B1**

(12) **EUROPÄISCHE PATENTSCHRIFT**

(45) Veröffentlichungstag der Patentschrift: **28.09.94**

(51) Int. Cl.5: **C07C 69/54**, C07C 67/48

(21) Anmeldenummer: **90900162.0**

(22) Anmeldetag: **15.12.89**

(86) Internationale Anmeldenummer:
**PCT/EP89/01547**

(87) Internationale Veröffentlichungsnummer:
**WO 90/07484 (12.07.90 90/16)**

(54) **VERBESSERTES TROCKENNEUTRALISATIONSVERFAHREN FÜR ORGANISCHE FLÜSSIGPHASEN.**

(30) Priorität: **24.12.88 DE 3843843**
**27.11.89 DE 3939163**

(43) Veröffentlichungstag der Anmeldung:
**09.10.91 Patentblatt 91/41**

(45) Bekanntmachung des Hinweises auf die
Patenterteilung:
**28.09.94 Patentblatt 94/39**

(84) Benannte Vertragsstaaten:
**BE CH DE ES FR GB IT LI NL**

(56) Entgegenhaltungen:
**DE-A- 1 493 004**
**FR-A- 2 316 253**
**FR-A- 2 358 377**

(73) Patentinhaber: **Henkel Kommanditgesellschaft auf Aktien**

**D-40191 Düsseldorf (DE)**

(72) Erfinder: **RITTER, Wolfgang**
**Am Bandenfeld 74**
**D-5657 Haan (DE)**
Erfinder: **SITZ, Hans-Dieter**
**Widdeshovener Strasse 48**
**D-4049 Rommerskirchen (DE)**
Erfinder: **SPEITKAMP, Ludwig**
**Tönisstrasse 13**
**D-4000 Düsseldorf 13 (DE)**

(74) Vertreter: **von Kreisler, Alek, Dipl.-Chem. et al**
**Patentanwälte**
**von Kreisler-Selting-Werner**
**Postfach 10 22 41**
**D-50462 Köln (DE)**

**Beschreibung**

Die Erfindung betrifft ein verbessertes Verfahren zur Reingewinnung von im wesentlichen neutralen schwerflüchtigen und Polymerisations- bzw. Vergelungs-gefährdeten organischen Komponenten, die reaktive olefinische Doppelbindungen aufweisen. Die Erfindung will dabei insbesondere eine verbesserte Möglichkeit zur Reingewinnung schwerflüchtiger organischer Komponenten der genannten Art schaffen, deren Reinigung auf destillativem Wege nicht oder nicht ohne weiteres möglich ist.

Die der Erfindung zugrunde liegende Problematik wird im nachfolgenden beispielhaft anhand einer ausgewählten Stoffklasse beschrieben, bei deren Herstellung die Mitverwendung der erfindungsgemäß vorgeschlagenen Maßnahmen besonders interessant ist. Für den Fachmann sofort einleuchtend gilt allerdings, daß der Umfang des Anwendungsgebietes der Erfindung sich nicht auf diese bestimmte Stoffklasse einschränkt. Die neu vorgeschlagenen Arbeitsmaßnahmen sind in beliebigen Anwendungsfällen vergleichbarer, technologisch vorgegebener oder frei gewählter Problemstellung einsetzbar.

Schwerflüchtige polyfunktionelle Ester der Acrylsäure und/oder der Methacrylsäure mit mehrwertigen Alkoholen - im folgenden auch als (Meth)acrylsäureester bzw. multifunktionelle (Meth)acrylsäureester bezeichnet - werden bekanntlich durch Umsetzung der Reaktanten in Gegenwart saurer Veresterungskatalysatoren unter Zusatz von Polymerisationsinhibitoren zum Reaktionsgemisch hergestellt. (Meth)acrylsäureester dieser Art finden zunehmende Bedeutung als hochreaktive Bestandteile in beispielsweise strahlenhärtenden Systemen. Die hier zugrunde liegenden polyfunktionellen Alkohole sind beispielsweise 2- bis 4-wertige aliphatische gesättigte Alkohole und/oder deren Oxalkylierungsprodukte. Polyfunktionelle (Meth)acrylsäureester der genannten Art können beispielsweise als Lackrohstoffe für die Elektronenstrahlhärtung oder als Bestandteil von UV-Licht-härtenden Druckfarben oder entsprechenden Oberzugslacken, in Spachteln, Form- oder Vergußmassen sowie in Klebstoffen, insbesondere anaerob härtenden Klebstoffen Verwendung finden. Ihre Herstellung ist allerdings nicht problemlos. Gefordert werden insbesondere möglichst farblose Produkte mit geringer Säurezahl und hoher Lagerstabilität, die praktisch auch keinen Eigengeruch aufweisen. Eine destillative Reinigung der (Meth)acrylsäureester der hier betroffenen Art scheidet in aller Regel aufgrund ihres hohen Molekulargewichtes und ihrer hohen Reaktivität aus. Die als Reaktionsrohprodukte anfallenden Stoffgemische, die lösungsmittelhaltig oder auch lösungsmittelfrei vorliegen, können den sauren Katalysator und gegebenenfalls auch Restsäureanteile aus der Veresterungsreaktion enthalten, müssen also einer abschließenden Neutralisation und Reinigungsbehandlung unterworfen werden, die das nichtdestillierte Reaktionsprodukt im geforderten Gütezustand liefert.

Der Erfindung liegt die Konzeption zugrunde, diese abschließende Neutralisation und Reinigungsstufe im Rahmen einer sogenannten Trockenneutralisation durchzuführen. Unter dem Begriff der Trockenneutralisation wird dabei die Arbeitsweise verstanden, die - bei wenigstens weitgehendem, bevorzugt vollständigem Ausschluß von Wasser - das organische als fließfähige Phase vorliegende Reaktionsrohprodukt mit einem wenigstens weitgehend trockenen basischen Neutralisationsmittel zur Bindung der sauren Komponenten (Katalysator, Restsäuren und dergleichen) versetzt wird, woraufhin nach Einstellung der angestrebten Rest-Säurezahl die zugesetzte Feststoffphase von der fließfähigen organischen Phase wieder abgetrennt wird.

Bei der Entwicklung eines technischen Verfahrens dieser Konzeption hat sich allerdings gezeigt, daß die Erfüllung des gestellten Anforderungsprofils an die Produkteigenschaften schwierig ist, weil bei vorgegebener Variation ausgewählter Verfahrensbedingungen zur Optimierung einer angestrebten Produkteigenschaft - beispielsweise der niedrigen Säurezahl - andere Produkteigenschaften, beispielsweise die Farbe und/oder die Stabilität des Reaktionsproduktes, negativ beeinflußt werden. Die komplexe Problematik wird beispielhaft aus dem folgenden ersichtlich: Hochreaktive (Meth)acrylatsysteme der geschilderten Art neigen zur Vergelung durch anionische Polymerisation bei Zusatz von feinteiligen festen basischen Neutralisationsmitteln. Die angestrebte Neutralisation der im Reaktionsgemisch vorliegenden sauren Komponenten wird gegenüber der anionischen Vergelung zur bevorzugten Reaktion, wenn die Reaktionstemperatur auf erhöhte Werte angehoben wird. Derart erhöhte Temperaturen wirken sich jetzt aber wieder nachteilig auf den sich einstellenden Restfarbwert des neutralisierten Gutes aus.

Von dieser sich gegenseitig behindernden Verknüpfung der angestrebten und einstellbaren Produkteigenschaften geht die Erfindung aus. Die erfindungsgemäße Lehre zeigt einen Weg, wie trotz dieser Behinderungen Endprodukte der gewünschten hochwertigen Qualifikation im gesamten Eigenschaftsprofil gewonnen werden können.

Mit der hier aufgezeigten und der erfindungsgemäßen Lehre zugrunde liegenden Problemstellung beschäftigt sich im druckschriftlichen Stand der Technik die Lehre der FR-A-2 358 377 und die zugehörige DE-B2 27 31 058. Die Lehre dieser Druckschriften geht von der Aufgabe aus, bei der Herstellung von (Meth)acrylsäureestern durch Umsetzung von (Meth)acrylsäure und Hydroxy-Verbindungen mit wenigstens einer primären Hydroxylgruppe in Gegenwart eines sauren Veresterungskatalysators, eines Polymerisationsinhibitors und eines mit Wasser nicht mischbaren organischen Lösungsmittels niedrigviskose Reaktionsprodukte herstellen zu können, die gleichzeitig eine niedrige Säurezahl und eine helle Farbe aufweisen. Zur technischen Lösung dieser Aufgabenstellung wird hier vorgeschlagen, daß man am Ende der Veresterungsreaktion eine Lithiumverbindung zu dem Veresterungsgemisch zufügt, um den sauren Veresterungskatalysator zu inaktivieren. Als Beispiele für geeignete Lithiumverbindungen sind Lithiumoxid und/oder Lithiumhydroxid ebenso wie Lithiumhalogenide, Lithiumsalze von Säuren, die einen pK-Wert aufweisen, der höher liegt als der des Veresterungskatalysators und damit eine Vielzahl von Lithiumsalzen benannt. Die Lithiumverbindungen können gegebenfalls Kristallwasser enthalten. Zugleich mit oder auch nach dem Zufügen der Lithiumverbindung(en) wird der verbliebene Überschuß an (Meth)acrylsäure durch Destillation entfernt. Der neutralisierte Veresterungskatalysator verbleibt offensichtlich im Reaktionsgemisch.

Die Lehre der FR-A-2 316 253 und der DE-A-1 493 004 bezieht sich auf die Herstellung ungesättigter Ester. Beschrieben werden im einzelnen Maßnahmen zur Entfernung der Versterungskatalysatoren und gegebenenfalls Inhibitoren durch Zugabe basischer Verbindungen insbesondere bei Raumtemperatur. Dabei können wässrige basische Lösungen eingesetzt werden, die auch zur Entfernung überschüssiger Acrylsäure zur Extraktion dienen sollen. Irgendwelche weitergehenden Hinweise auf die im nachfolgenden mit der Erfindungsbeschreibung geschilderte Verknüpfung erfindungswesentliche Parameter zur Lösung der eingangs dargestellten erfindungsgemäßen Aufgabe finden sich in diesen Druckschriften nicht.

## Die erfindungsgemäße Lehre

Gegenstand der Erfindung ist demgegenüber ein Verfahren zur Reingewinnung von im wesentlichen neutralen, schwerflüchtigen und reaktive olefinische Doppelbindungen aufweisenden organischen Verbindungen aus Einsatzmaterialien, die diese Komponenten zusammen mit untergeordneten Mengen saurer Reaktionsbestandteile und/oder entsprechender Hilfsstoffe in Flüssigphase enthalten mittels Neutralisation und Abtrennung der gebildeten Salze, dadurch gekennzeichnet, daß man zur Gewinnung von Reinprodukten, die auch ohne Destillation niedrige Rest-Säurezahlen mit niedrigen Farbzahlen verbinden, die Neutralisation mit festen feinpulvrigen Oxiden, Carbonaten und/oder Hydroxiden der Alkali- und/oder Erdalkalimetalle bei Temperaturen oberhalb 50 °C als Trockenneutralisation durchführt, dabei wenigstens zeitweise unter verringertem Druck und Abziehen auch des Neutralisationswassers arbeitet und nachfolgend die organische Flüssigphase von der feinpulvrigen Feststoffphase abtrennt.

Besondere Bedeutung kommt der Verwendung von feinteiligen, insbesondere feinpulvrigen Oxiden und/oder Hydroxiden von Calcium und/oder Magnesium zur Durchführung der Trockenneutralisation zu. Hierbei gilt, daß von diesen beiden Erdalkalimetallen den entsprechenden Verbindungen des Calciums wiederum besondere Bedeutung zukommt. In einer bevorzugten Ausführungsform der Erfindung werden feinteilige Feststoffe in die Trockenneutralisationsstufe eingeführt, die wenigstens anteilsweise Calciumverbindungen der genannen Art enthalten. In einer besonders wichtigen Ausführungsform wird Calciumhydroxid $Ca(OH)_2$ und/oder gebrannter Kalk $CaO$ eingesetzt. Insbesondere feinteiliges Calciumhydroxid ermöglicht häufig eine optimal ausgleichende Einstellung der sich eigentlich gegensätzlich auswirkenden Verfahrensparameter bezüglich Restsäurezahl, Farbzahl, thermischer Belastbarkeit, Verfahrensdauer, Menge des einzusetzenden trockenen Neutralisationsmittels und dergleichen.

Ein weiterer wichtiger Parameter, der in der bisherigen Erfindungsschilderung nicht angesprochen worden ist, ist die mögliche Interaktion zwischen dem eingesetzten Trockenneutralisationsmittel und den üblicherweise in den hier betroffenen eingesetzten Systemen mitverwendeten Polymerisationsinhibitoren. Auch hier sind - bestimmt durch die spezielle Struktur des im Einzelfall vorliegenden Inhibitors bzw. Inhibitorsystems einerseits und des eingesetzten Neutralisationsmittels im Festzustand andererseits - Interaktionen möglich, wenn auch nicht zwingend. Einzelheiten hierzu finden sich in der parallelen Patentanmeldung (EP-90 917 724.8) der Anmelderin. Im Rahmen des Arbeitens im Sinne der in der vorliegenden Erfindungsoffenbarung beschriebenen technischen Lehre wird der nacharbeitende Fachmann von Fall zu Fall zu prüfen haben, in welchem Ausmaß die Inhibierung des trockenneutralisierten Reaktionsproduktes nach Abtrennung der Feststoffphase sichergestellt ist. Gegebenenfalls wird der Inhibitorgehalt des gereinigten Reaktionsproduktes auf den vorbestimmten Wert durch Zugabe zusätzlicher Inhibitoranteile eingestellt werden müssen. Grundsätzlich gilt, daß auch Inhibitoren der hier in Betracht kommenden üblichen Stoffklassen zur Inhibierung einer unerwünschten radikalischen Polymerisationsauslösung mehr oder weniger stark mit den Trockenneutralisationsmitteln in Interaktion treten und über diese aus dem Reaktionsgemisch - im allgemeinen anteilsweise - entfernt werden können. Je nach den an das Endprodukt zu stellenden Anforderungen bezüglich der Inhibierung kann dann ein Ersatz ausgetragener Anteile der Radikalinhibitoren zweckmäßig oder auch notwendig sein.

Ein erster wesentlicher Verfahrensparameter für die erfindungsgemäße Optimierung ist die Festlegung des Temperaturbereichs für die Trockenneutralisation. Auch die erfindungsgemäß ausgewählten Feststoffsysteme auf Basis der Oxide und/oder Hydroxide der Erdalkalimetalle, insbesondere des Magnesiums und/oder Calciums können bei vergleichsweise hochreaktiven olefinisch ungesättigten Systemen im Bereich der Raumtemperatur oder auch noch bei schwach erhöhten Temperaturen zur Auslösung einer anionischen Polymerisation und damit zur Vergelung des Reaktionsproduktes führen. Erfindungsgemäß ist es dementsprechend vorgesehen die Trockenneutralisation bei Temperaturen oberhalb 50 °C durchzuführen, wobei sich als besonders interessanter Temperaturbereich die Spanne von etwa 60 bis 100 °C und insbesondere der Bereich von etwa 70 bis 90 °C erwiesen haben. Temperaturen dieses Arbeitsbereichs bevorzugen die salzbildende Neutralisation gegenüber der anionischen Polymerisationsauslösung. Die Gefahr der unerwünschten Farbvertiefung im Reaktionsprodukt ist allerdings durch diese Temperaturerhöhung beträchtlich verstärkt.

Zur Bewältigung dieses Problemkreises dient einerseits die erfindungsgemäße vorgesehene eingeschränkte Auswahl der Trockenneutralisationsmittel. Zusätzlich kann hier Einfluß auf die Farbe durch Festlegung der Dauer der Neutralisationsbehandlung genommen werden. Dabei gilt allerdings das folgende: Die erfindungsgemäß vorgesehene Trockenneutralisation verläuft im Vergleich mit der Salzbildung aus Ionenlösungen langsam. Diese Zeitverzögerung in der Neutralisation wird durch zwei hier zu berücksichtigende Effekte zusätzlich begünstigt: Aus Gründen der Wirtschaftlichkeit, aber auch aus Gründen des optimalen Schutzes des an sich labilen Reaktionsgemisches wird das feste basische Neutralisationsmittel vorzugsweise nur in beschränktem stöchiometrischen Überschuß eingesetzt. Die hinreichende Verweildauer des Neutralisationsmittels in Suspension im flüssigen Einsatzgut muß also ausreichend lang gewählt werden, um den hinreichenden Kontakt zwischen festen und flüssigen bzw. gelösten Reaktanten sicherzustellen. Darüber hinaus belegt sich die Außenfläche des individuellen Feststoffpartikels vergleichsweise rasch mit dem jeweils entstehenden Salz. Das tiefere Eindringen der zu neutralisierenden sauren Komponenten in den Kern des Feststoffteilchens wird damit behindert.

Es ist möglich, die Trockenneutralisation im Sinne der Erfindung jeweils über einen Zeitraum von einigen Stunden, beispielsweise bis zu 3 Stunden, vorzugsweise bis zu 1 oder 2 Stunden durchzuführen. Für die Optimierung im Sinne des erfindungsgemäßen Verfahrens kann es allerdings zweckmäßig sein, Verfahrenstemperatur und Verfahrensdauer so aufeinander abzustimmen, daß zur Einstellung der erwünschten niederen Restsäurezahlen eine Behandlungsdauer unterhalb 1 Stunde und insbesondere unterhalb 45 Minuten möglich wird. In besonders bevorzugten Ausführungsformen werden die Arbeitsbedingungen so aufeinander abgestimmt, daß Restsäurezahlen unterhalb 1 mg KOH/g Substanz im Zeitraum bis etwa 30 Minuten und vorzugsweise im Zeitraum bis etwa 5 bis 30 Minuten eingestellt werden. Beim Arbeiten mit Calciumhydroxid als Trockenneutralisationsmittel können in der Regel optimale Werte im Temperaturbereich um ca. 80 °C einer Behandlungsdauer von etwa 5 bis 60 Minuten eingestellt werden. Erfindungsgemäß gelingt es damit, die geforderten niederen Restsäurezahlen einzustellen, die bevorzugt bei Werten von etwa 0,5 mg KOH/g Substanz oder auch noch darunter liegen, gleichzeitig aber die Farbzahlen des nicht destillierten Gutes im Bereich unterhalb 2 und vorzugsweise im Bereich von etwa 1 zu halten. Die Bestimmung der Farbzahlen erfolgt dabei gemäß GARDNER.

Wichtige Bedeutung kann insbesondere für die Entwicklung der Farbzahl im Endprodukt dem nachfolgend geschilderten Parameter des erfindungsgemäßen Verfahrens zukommen:

Es hat sich gezeigt, daß die Gegenwart von Wasser im zu behandelnden Reaktionsgut die Entwicklung der einstellbaren Farbzahl negativ beeinflussen kann. Auch vergleichsweise geringe Mengen wäßriger Basenlösungen der hier betroffenen Art wirken offenbar unter den ansonsten optimierten Verfahrensbedingungen stärker verfärbend als das trockene Neutralisationsmittel in Feststoff-Form. Erfindungsgemäß ist dementsprechend zunächst einmal bevorzugt, das zu neutralisierende und als Flüssigphase einzusetzende Gut praktisch wasserfrei vorzulegen. Gegebenenfalls im zu reinigenden Gut vorliegende Restwassermengen können in an sich bekannter Weise beispielsweise unter Mitverwendung von Hilfslösungsmitteln in azeotroper Destillation oder einfach durch Anlegen von Vakuum für einen hinreichend langen Zeitraum abgezogen werden.

Gemäß der Erfindung wird diesem Gedanken aber auch in der folgenden Weise nachgegangen: Es hat sich alserforderlicherwiesen, die Neutralisationsreaktion selbst dann wenigstens zeitweise unter verringertem Druck durchzuführen, wenn das eingesetzte zu neutralisierende Flüssiggut an sich wasserfrei ist. Bei der Arbeitsweise der Neutralisation unter verringertem Druck wird auch der im Rahmen der Neutralisation entstehende Wasseranteil aus dem Reaktionsgemisch abgezogen und damit die in situ-Bildung wäßrig-basischer Lösungsanteile bei der Neutralisation verhindert oder zumindest eingeschränkt. Es kann hier zweckmäßig sein, die Neutralisation im Druckbereich von etwa 1 bis 150 mbar, z. B. bei etwa 20 bis 150 mbar durchzuführen, wobei das Arbeiten im Bereich von etwa 20 bis 100 mbar besonders bevorzugt sein kann. Auf diese Weise gelingt es, Restwassergehalte im Fertigprodukt auf Werte unter etwa 0,1 Gew.-% abzusenken, während bei der Trockenneutralisation praxisüblicher Rohprodukte aufgrund ihres Gehaltes an freien Säurekomponenten nach Abschluß der Neutralisation ohne Anlegen von Vakuum Restwassergehalte im Bereich von etwa 0,7 bis 0,9 Gew.-% liegen können. Es hat sich gezeigt, daß schon durch diese höheren Restwasser-Gehalte substantielle Verschlechterungen in der Farbzahl des gereinigten Endproduktes ausgelöst werden können.

Die Menge des in der Neutralisationsstufe zugesetzten feinpulvrigen basischen Hilfsstoffes wird primär durch die insgesamt zu neutralisierende Menge an freien Säurekomponenten im Rohprodukt bestimmt. Vorzugsweise werden die feinpulvrigen Neutralisationsmittel in wenigstens etwa stöchiometrischer Menge zugegeben. Es kann in der Regel zweckmäßig sein, mit einem beschränkten Überschuß des festen Neutralisationsmittels zu arbeiten. Im allgemeinen wird der Überschuß das etwa 2,5- bis 3-fache der stöchiometrisch benötigten Menge - wiederum bezogen auf insgesamt vorliegende Säure - nicht überschreiten, wobei das Arbeiten mit Basenmengen in dem Bereich des etwa 1,3- bis 2-fachen der stöchiometrisch benötigten Menge besonders geeignet sein kann.

Es hat sich gezeigt, daß den erfindungsgemäß zur Neutralisation vorgesehenen Komponenten in der Feinabstimmung ihrer jeweils ausgelösten Wirkungen Individuelles zukommen kann. Pulverförmiges Calciumhydroxid senkt sehr wirkungsvoll die Restsäurezahl und kann auch zu niedrigen Werten in der Farbzahl führen. Das letzte ist allerdings nur dann gewährleistet, wenn die Kontaktzeit des Reaktionsgutes mit dem Calciumhydroxid vergleichsweise kurz gehalten wird. Wie bereits angegeben, kann hier das Arbeiten im Temperaturbereich von etwa 80 °C für eine Zeitdauer von etwa 10 bis 30 Minuten zu optimalen Ergebnissen führen. Calciumhydroxid hat auch eine vergleichsweise starke Wirkung bezüglich der im Reaktionsgut verbleibenden Restanteile an Polymerisationsinhibitor - hinreichende Reaktivität zwischen dem Inhibitor und Calciumhydroxid vorausgesetzt. Demgegenüber ist der gebrannte Kalk ein gutes Neutralisationsmittel, der allerdings eine vergleichsweise geringere Interaktion mit an sich zur Salzbildung befähigten Inhibitoren zeigt. Die Kombination von Magnesium- und Calciumverbindungen der genannten Art kann zu verbesserten Farbzahlen bei gleichzeitig hinreichender Neutralisationswirkung führen. Auch hiermit kann gleichzeitig eine vergleichsweise schwache Interaktion mit zur Salzbildung befähigten Inhibitoren verbunden sein.

In einer Ausführungsform , können die erfindungsgemäß ausgewählten oxidischen bzw. hydroxidischen Erdalkaliverbindungen auch in Abmischung mit Hydrotalcit zum Einsatz kommen. Kombinationen aus Hydrotalcit und basischen Calciumverbindungen der genannten Art führen bei guter Absenkung der Restsäurezahl zu besonders guten Farbzahlen im Fertigprodukt.

Hydrotalcite sind bekanntlich in feinteiliger unlöslicher Form herstellbare Mischhydroxidverbindungen anorganischer Grundstruktur, die 2-dimensionale anorganische Polykationen mit innerkristallinem Ladungsausgleich durch bewegliche Zwischenschicht-Anionen darstellen und auch unter der Bezeichnung "Doppelschichthydroxide" bekannt und mehrfach in der Literatur beschrieben sind, verwiesen wird zum Beispiel auf R. Allmann, "Doppelschichtstrukturen mit brucitähnlichen Schichtionen ..." Chimia 24, 99 bis 108 (1970). Verschiedene Möglichkeiten zur technischen Herstellung dieser Verbindungen werden in der DE-OS 20 61 156 angegeben. Ein gut charakterisierter Vertreter dieser Stoffgruppe ist der als Mineral in der Natur vorkommende aber auch synthetisch herstellbare Hydrotalcit, ein Magnesium-Aluminium-Hydroxocarbonat der ungefähren Zusammensetzung $Mg_6Al_2(OH)_{16} \cdot CO_3 \times 4\ H_2O$. Vergleiche R. Allmann et al. "Die Struktur des Hydrotalkits" N. Jahrb. Mineral. Monatsh. 1969, 544 bis 551.

Die nachfolgenden Beispiele beschreiben die Wirkung verschiedenartiger Trockenneutralisationsmittel im Sinne der erfindungsgemäßen Definition, ihre Einzelwirkungen bei Variation der Verfahrensparameter sowie optimale Ergebnis-Kombinationen bei geeigneter Abstimmung der erfindungsgemäßen Verfahren bestimmenden Elemente im Sinne der hier gegebenen Lehre. Soweit diese Beispiele unter Normaldruck arbeiten - Beispiele 1, 2 (zum Teil) und 6 - fallen sie nicht unter die beanspruchte Elementenkombination der Erfindung, gleichwohl geben sie dem Fachmann Hinweise auf die Bedeutung einzelner Parameter des erfindungsgemäßen Handeln.

**Beispiele**

Beispiel 1

In einem 1-Liter-Kolben wurden jeweils 800 g eines Veresterungsrohproduktes - bestehend aus 28 g Toluolsulfonsäure, 1,6 g 2,5-Di-tert.-butylhydrochinon, 30,8 g Acrylsäure und 739,6 g Trimethylolpropan + 3 EO-triacrylat - eingewogen. Die Neutralisation des Rohproduktes erfolgte bei Temperaturen von 80 bis 40 °C unter Rühren und Durchleiten von 10 l Luft/h. Zur Neutralisation wurde die zweifach äquivalente Menge an Base - bezogen auf die Säurezahl des Rohproduktes (Säurezahl: 40 mg KOH/g Substanz) - eingesetzt.

Nach 1, 2, 3 und 24 Stunden wurden jeweils ca. 200 g des neutralisierten Produktes entnommen und mittels Druckfilternutsche filtriert. Die Ergebnisse bezüglich der Produktsäurezahlen sind in der Tabelle 1 zusammengefaßt.

Nur mit den Basen, die bei einer Neutralisationstemperatur von 80 °C Säurezahlen kleiner gleich 6 mg KOH/g lieferten, wurde eine Temperaturreduzierung auf 60 °C durchgeführt. Die Basen, die dann zu Säurezahlen kleiner gleich 6 mg KOH/g führten, wurden auch zur Neutralisation bei 40 °C eingesetzt.

Tabelle 1: Screening der Neutralisationssubstanzen hinsichtlich der Säurezahl

Ausgangsprodukt: Trimethylolpropan + 3 EO-triacrylat (Säurezahl: 40 mg KOH/g Substanz)

| Base | Neutralisation: 2fach äqui-molar; 80 $^{\circ}$C | | | | Neutralisation: 2fach äqui-molar; 60 $^{\circ}$C | | | | Neutralisaion: 2fach äqui-molar; 40 $^{\circ}$C | | | |
|---|---|---|---|---|---|---|---|---|---|---|---|---|
| | 1 h | 2 h | 3 h | 1 d | 1 h | 2 h | 3 h | 1 d | 1 h | 2 h | 3 h | 1 d |
| | mg/g | mg/g | mg/g | mg/g | mg/g | mg/g | mg/g | mg/g | mg/g | mg/g | mg/g | mg/g |
| Li2CO3 (MG: 73,9) | | | | | | | | | | | | |
| LiOH (MG: 24,0) | 9,8 | 2,2 | 1,3 | 1,3 | 17,6 | 14,3 | 10,5 | 9,2 | | | | |
| Na2CO3 (MG:106,0) | 4,6 | – | 0,4 | 2,6 | 2,5 | 1,2 | 0,4 | 0,2 | 29,3 | 25,6 | 7,8 | ver-gelt |
| NaHCO3 (MG: 84,0) | 31,6 | 26,2 | 23,7 | 27,1 | | | | | | | | |
| NaOH fein (MG: 40,0) | 25,8 | 5,0 | 2,8 | 2,4 | 9,4 | 0,7 | 0,4 | 0,2 ver-gelt | 35,7 | 29,8 | 27,3 | 25,5 ver-gelt |
| NaOH grob (MG: 40,0) | 38,4 | 36,4 | 33,2 | 34,2 | | | | | | | | |

EP 0 449 865 B1

| Base | Neutralisation: 2fach äqui-molar; 80 °C | | | | Neutralisation: 2fach äqui-molar; 60 °C | | | | Neutralisaion: 2fach äqui-molar; 40 °C | | | |
|---|---|---|---|---|---|---|---|---|---|---|---|---|
| | 1 h mg/g | 2 h mg/g | 3 h mg/g | 1 d mg/g | 1 h mg/g | 2 h mg/g | 3 h mg/g | 1 d mg/g | 1 h mg/g | 2 h mg/g | 3 h mg/g | 1 d mg/g |
| $K_2CO_3$ (MG:138,2) | 6,7 | 2,1 | 0,7 | 1,1 | 7,9 | 1,9 | 0,2 | 0,5 | 10,3 | 7,9 | 1,9 ver-gelt | vergelt |
| $KHCO_3$ (MG:100,1) | | | | | | | | | | | | |
| KOH fein (MG: 56,1) | 39,1 | 29,6 | 24,4 | 22,3 | | | | | | | | |
| KOH grob (MG: 56,1) | 38,7 | 37,9 | 35,8 | 36,2 | | | | | | | | |
| $(MgCO_3)4$ x $Mg(OH)_2$ x $5H_2O$ (MG:485,7) | 27,9 | 24,6 | 25,3 | 29,0 | | | | | | | | |
| $Mg(OH)_2$ (MG: 58,3) | 15,1 | 10,9 | 8,5 | 14,4 | 30,0 | 28,7 | 26,3 | 26,5 | | | | |
| MgO (MG: 40,3) | 5,8 | 5,6 | 3,1 | 5,7 | 11,3 | 10,1 | 7,4 | 10,0 | | | | |

EP 0 449 865 B1

| Base | Neutralisation: 2fach äqui-molar; 80 °C | | | | Neutralisation: 2fach äqui-molar; 60 °C | | | | Neutralisaion: 2fach äqui-molar; 40 °C | | | |
|---|---|---|---|---|---|---|---|---|---|---|---|---|
| | 1 h mg/g | 2 h mg/g | 3 h mg/g | 1 d mg/g | 1 h mg/g | 2 h mg/g | 3 h mg/g | 1 d mg/g | 1 h mg/g | 2 h mg/g | 3 h mg/g | 1 d mg/g |
| CaCO3 (MG:100,0) | 29,3 | 27,8 | 26,4 | 29,0 | | | | | | | | |
| Ca(OH)2 (MG: 74,1) | 0,9 | 0,2 | 0,1 | 0,3 | 5,1 | 0,7 | 0,3 | 0,5 | 4,2 | ver-gelt | | |
| CaO (MG: 56,1) | 23,6 | 8,4 | 3,2 | 0,6 | 23,6 | 17,7 | 12,9 | 9,0 | | | | |
| Al2O3 (MG: 102,0) | 39,7 | 39,7 | 37,4 | 37,2 | | | | | | | | |
| Al(OH)3 (MG: 78,0) | 39,8 | 37,8 | 36,5 | 40,2 | | | | | | | | |
| Hydrotalcit (MG: 604) | 7,7 | 6,8 | 5,6 | 6,1 | 18,9 | 17,3 | 15,0 | 15,6 | | | | |

Beispiel 2

1,5 kg eines Veresterungsrohproduktes (52,5 g p-Toluolsulfonsäure, 3,0 g 2,5-Di-tert.-butylhydrochinon, 53,9 g Acrylsäure und 1390,6 g Trimethylolpropan + 3 EO-triacrylat, OH-Zahl 34 mg KOH/g, Säurezahl 38 mg KOH/g, Gardner Farbzahl 1, $H_2O$-Gehalt 0,40 Gew.-%) wurden in einen 2-Liter-Reaktor eingewogen und bei 80 °C unter Durchleiten von 20 l Luft/h mit 48,9 g $Ca(OH)_2$ neutralisiert. Nach 1, 2 und 3 Stunden Neutralisationsdauer wurde jeweils 1/3 der Produktmenge entnommen und mittels Druckfilternutsche filtriert. Die Neutralisation wurde zum einen bei Normaldruck durchgeführt und zum anderen bei 50 mbar Vakuum zur Eliminierung von Wasserresten. Die Ergebnisse sind in Tabelle 2 zusammengefaßt.

## Tabelle 2

## Abhängigkeit der Produktspezifikationen vom Druck während der Neutralisation

Ausgangsprodukt: Trimethylolpropan + 3 EO-triacrylat

Säurezahl: 38 mg KOH/g

Hydroxylzahl: 34 mg KOH/g

Gardner Farbzahl: 1

Wassergehalt: 0,40 Gew.-%

| Temperatur: 80 °C | Neutrali- sationszeit h | Säure- zahl mg KOH/g | Hydroxyl- zahl mg KOH/g | Farb- zahl Gardner | Wasser- gehalt Gew.-% |
|---|---|---|---|---|---|
| 48,9 g $Ca(OH)_2$ Normaldruck | 1 | 2,9 | 50 | kleiner 1 | 0,90 |
| | 2 | 0 | 51 | 2 | 0,83 |
| | 3 | 0 | 51 | 3 | 0,61 |
| 48,9 g $Ca(OH)_2$ 50 mbar | 1 | 7,1 | 47 | kl. 1 | 0,06 |
| | 2 | 4,1 | 47 | kl. 1 | 0,10 |
| | 3 | 2,0 | 48 | kl. 1 | 0,02 |

Beispiel 3

1,0 kg eines Veresterungsproduktes (analog Beispiel 2) wurde in einen 2-Liter-Reaktor eingewogen und bei einer Temperatur von 80 °C und einem Druck von 50 mbar unter Durchleiten von 20 l Luft/h neutralisiert. Die Mengen an Neutralisationsmitteln betrug dabei:
$Ca(OH)_2$ 26,2 g; 32,5 g; 34,5 g; 37,5 g; 39,9 g; 49,9 g
Ca O 28,4 g; 37,8 g
Nach 1, 2 und 3 Stunden Neutralisationszeit wurde jeweils 1/3 der Produktmenge entnommen und mittels Druckfilternutsche filtriert. Die Ergebnisse bezüglich Säurezahl, OH-Zahl und Farbzahl sind in Tabelle 3 zusammengefaßt.

Tabelle 3

Abhängigkeit der Produktspezifikationen von der Neutralisationsmittelmenge

Ausgangsprodukt: Trimethylolpropan + 3 EO-triacrylat

Säurezahl: 38 mg KOH/g

Hydroxylzahl: 34 mg KOH/g

Gardner Farbzahl: 1

Wassergehalt: 0,40 Gew.-%

| Temperatur: 80 $^{\circ}$C Druck:50mbar | Neutralisa- tionszeit h | Säurezahl mg KOH/g | Hydroxyl- mg KOH/g | Farbzahl Gardner |
|---|---|---|---|---|
| 26,2 g Ca(OH)$_2$ | 1 | 9,2 | 39,7 | kl. 1 |
| 1fach äquival. | 2 | 6,7 | 42,8 | kl. 1 |
| | 3 | 5,7 | 48,8 | kl. 1 |
| 32,5 g Ca(OH)$_2$ | 1 | 7,1 | 47 | kl. 1 |
| 1,3fach äquival. | 2 | 4,1 | 47 | kl. 1 |
| | 3 | 2,0 | 48 | kl. 1 |
| 34,5 g Ca(OH)$_2$ | 1 | 5,1 | 50 | kl. 1 |
| 1,4fach äquival. | 2 | 2,6 | 50 | kl. 1 |
| | 3 | 0,2 | 50 | 2 |
| 37,5 g Ca(OH)$_2$ | 1 | 2,7 | 45 | kl. 1 |
| 1,5fach äquival. | 2 | 1,5 | 45 | 1 |
| | 3 | 0,4 | 53 | 2 |
| 39,9 g Ca(OH)$_2$ | 1 | 2,9 | 46 | 1 |
| 1,6fach äquival. | 2 | 0 | 46 | 3 |
| | 3 | 0 | 49 | 4 |
| 49,9 g Ca(OH)$_2$ | 1 | 0 | 48 | 1 |
| 2fach äquival. | 2 | 0 | 51 | 2 |
| | 3 | 0 | 54 | 3 |
| 28,4 g CaO | 1 | 11,4 | 43 | kl. 1 |
| 1,5fach äquival. | 2 | 6,5 | 44 | kl. 1 |
| | 3 | 4,8 | 46 | kl. 1 |
| 28,4 g CaO | 1 | 3,9 | 44 | kl. 1 |
| 2fach äquival. | 2 | 1,7 | 49 | 1 - 2 |
| | 3 | 0,1 | 49 | 1 - 2 |

Beispiel 4

1,8 kg eines Veresterungsproduktes (analog Beispiel 2) wurden in einen 2-Liter-Reaktor eingewogen und bei 80 °C und 50 mbar unter Durchleiten von 20 l Luft/h mit 90,3 Ca(OH)$_2$ neutralisiert.

Nach 10, 20, 30, 40, 50, 60, 120, 180 und 1440 Minuten wurden jeweils ca. 200 g an Produkt entnommen und mittels Druckfilternutsche filtriert. Die Ergebnisse bezüglich Säurezahl, Hydroxyl- und und Farbzahlen sind in der Tabelle 4 aufgeführt.

## Tabelle 4

Abhängigkeit der Produktspezifikationen von der Neutralisations-zeit

Ausgangsprodukt: Trimethylolpropan + 3 EO-triacrylat

Säurezahl: 38 mg KOH/g

Hydroxylzahl: 34 mg KOH/g

Gardner Farbzahl: 1

Wassergehalt: 0,40 Gew.-%

| Temperatur: 80 °C Druck:50mbar | Neutralisa-tionszeit min. | Säurezahl mg KOH/g | Hydroxyl- mg KOH/g | Farbzahl Gardner |
|---|---|---|---|---|
| 90,3 g Ca(OH)$_2$ 2fach äquival. | 10 | 0,3 | 39 | kl. 1 |
| | 20 | 0 | 42 | kl. 1 |
| | 30 | 0 | 45 | kl. 1 |
| | 40 | 0 | 47 | kl. 1 |
| | 50 | 0 | 47 | 1 |
| | 60 | 0 | 48 | 1 |
| | 120 | 0 | 51 | 2 |
| | 180 | 0 | 54 | 3 |
| | 1440 | 0 | 58 | 4 |

Beispiel 5

1,0 kg eines Veresterungsproduktes (35 g p-Toluolsulfonsäure; 2 g 2,5-Di-tert.-butylhydrochinon; 38,5 g Acrylsäure, 924,5 g Neopentylglykol + 2 PO-diacrylat; Hydroxylzahl: 20 mg KOH/g, Säurezahl 40 mg KOH/g, Gardner Farbzahl 1) wurden in einen 2-Liter-Reaktor eingewogen und bei 80 °C und 50 mbar unter Durchleiten von 20 l Luft/h neutralisiert. Bei den Neutralisationsmitteln handelt es sich um Mischungen bzw. Kombinationsanwendungen von schwächer und stärker basischen Substanzen.

EP 0 449 865 B1

Mischungen (1 : 1 äquivalent):

| a) | 10,2 g MgO<br>18,7 g Ca(OH)$_2$ | (1,5fach äquivalent bezogen auf die Rohproduktsäurezahl) |
|---|---|---|
| b) | 17,8 g Hydrotalcit<br>19,7 g Ca(OH)$_2$ | (1,5fach äquivalent bezogen auf die Rohproduktsäurezahl) |

Kombinationen:

| a) | 28,5 g MgO 60 min.<br>+ 20,1 g Ca(OH)$_2$ 120 min. | (2fach äquivalent bezogen auf die Säurezahl des Rohproduktes)<br>(1,5fach äquivalent bezogen auf die Säurezahl nach 60 min.) |
|---|---|---|
| b) | 47,5 g Hydrotalcit 60 min.<br>+ 8,0 g Ca(OH)$_2$ 120 min. | (2fach äquivalent bezogen auf die Säurezahl des Rohproduktes)<br>(1,5fach äquivalent bezogen auf die Säurezahl nach 60 min.) |

Nach 60, 120 und 180 Minuten wurde jeweils 1/3 der Produktmenge entnommen und mittels Druckfilternutsche filtriert. Die Ergebnisse bezüglich Säure-, Hydroxyl- und Farbzahl sind in der Tabelle 5 zusammengefaßt.

## Tabelle 5
### Abhängigkeit der Produktspezifikationen bei Verwendung von Mischungen und Kombinationen von Neutralisationssubstanzen

Ausgangsprodukt: Neopentylglykol + 2 PO-diacrylat

Säurezahl: 40 mg KOH/g

Hydroxylzahl: 20 mg KOH/g

Gardner Farbzahl: 1

13

| Temperatur: 80 °C Druck:50mbar | Neutralisa- tionszeit min. | Säurezahl mg KOH/g | Hydroxyl- mg KOH/g | Farbzahl Gardner |
|---|---|---|---|---|
| Mischung: | | | | |
| 1:1 äquival. | 60 | 3,7 | 36 | kl. 1 |
| 18,7 g Ca(OH)$_2$ | | | | |
| 10,2 g MgO | 120 | 1,9 | 38 | kl. 1 |
| (1,5fach | | | | |
| äquival.) | 180 | 1,6 | 42 | kl. 1 |
| Mischung: | | | | |
| 1:1 äquival. | 60 | 4,2 | 35 | kl. 1 |
| 19,7 g Ca(OH)$_2$ | | | | |
| 17,8 g Hydro- | 120 | 2,8 | 35 | kl. 1 |
| talcit | | | | |
| (1,5fach | 180 | 1,9 | 39 | kl. 1 |
| äquival.) | | | | |
| Kombination | | | | |
| 28,5 g MgO | 60 | 20,2 | 31 | kl. 1 |
| (2fach äquiv. | | | | |
| bez. auf SZ) | | | | |
| + 20 g Ca(OH)$_2$ | 120 | 0,7 | 37 | kl. 1 |
| (1,5fach äqui- | | | | |
| val. bez. auf | 180 | 0,3 | 40 | kl. 1 |
| SZ nach 60min.) | | | | |
| Kombination | | | | |
| 47,5 g Hydro- | 60 | 8,1 | 35 | kl. 1 |
| talcit (2fach | | | | |
| äquival. bez. | 120 | 1,4 | 37 | kl. 1 |
| auf SZ) | | | | |
| + 8,0g Ca(OH)$_2$ | 180 | 0,8 | 40 | kl. 1 |
| (1,5fach äqui- | | | | |
| val. bez. auf | | | | |
| SZ nach 60min.) | | | | |

Beispiel 6

In einem 2-Liter-Reaktor wurden jeweils 1551,7 g eines Veresterungsproduktes - bestehend aus 43,4 g p-Toluolsulfonsäure, 2,5 g 2,5-Di-tert.-butylhydrochinon, 36,5 g Acrylsäure, 1119,1 g Trimethylolpropan + 3 EO-triacrylat und 350,2 g Toluol (Säurezahl: 18 mg KOH/g; Hydroxylzahl 25,3 mg KOH/g; Gardner Farbzahl: kleiner 1) - eingewogen.

Die Neutralisation wurde bei 80 °C und Normaldruck durchgeführt. Als Neutralisationsmittel wurden 36,9 g $Ca(OH)_2$; 27,9 g CaO sowie Kombinationen aus 20,1 g MgO (60 min.) + 28,9 g $Ca(OH)_2$ (120 min.) und 25,6 g Hydrotalcit (60 min.) + 19,9 g $Ca(OH)_2$ (120 min.) eingesetzt. Nach 1, 2 und 3 Stunden wurde jeweils 1/3 der Produktmenge entnommen und mittels Druckfilternutsche filtriert. Die Ergebnisse bezuglich Säure-, Hydroxyl- und Farbzahlen sind in der Tabelle 6 zusammengefaßt.

## Tabelle 6

## Abhängigkeit der Produktspezifikationen bei Verwendung von Lösungsmittel

**Ausgangsprodukt: Trimethylolpropan + 3 EO-triacrylat in Toluol**

**Säurezahl: 18 mg KOH/g**

**Hydroxylzahl: 25 mg KOH/g**

**Gardner Farbzahl: kleiner 1**

| Temperatur: 80 °C Druck:50mbar | Neutralisa- tionszeit min. | Säurezahl mg KOH/g | Hydroxyl- mg KOH/g | Farbzahl Gardner |
|---|---|---|---|---|
| 36,9 g Ca(OH)$_2$ | 60 | 1,3 | 26 | kl. 1 |
| (2fach äquival.) | 120 | 0,5 | 26 | 2 |
| | 180 | 0,4 | 28 | 4 |
| 27,9 g CaO | 60 | 5,6 | 27 | kl. 1 |
| (2fach äquival.) | 120 | 4,0 | 27 | kl. 1 |
| | 180 | 2,7 | 28 | kl. 1 |
| Kombination | | | | |
| 20,1 g MgO | 60 | 14,1 | 25 | kl. 1 |
| (2fach äquiv. bez. auf SZ) | | | | |
| + 28,9g Ca(OH)$_2$ | 120 | 2,6 | 25 | kl. 1 |
| (1,5fach äqui- | | | | |
| val. bez. auf | 180 | 0,5 | 26 | kl. 1 |
| SZ nach 60min.) | | | | |
| Kombination | | | | |
| 25,6 g Hydro- | 60 | 9,7 | 25 | kl. 1 |
| talcit (2fach | | | | |
| äquival. bez. | 120 | 2,6 | 25 | kl. 1 |
| auf SZ) | | | | |
| + 19,9g Ca(OH)$_2$ | 180 | 1,1 | 26 | kl. 1 |
| (1,5fach äqui- | | | | |
| val. bez. auf | | | | |
| SZ nach 60min.) | | | | |

**Patentansprüche**

1. Verfahren zur Reingewinnung von im wesentlichen neutralen, schwerflüchtigen und reaktive olefinische Doppelbindungen aufweisenden organischen Verbindungen aus Einsatzmaterialien, die diese Komponenten zusammen mit untergeordneten Mengen saurer Reaktionsbestandteile und/oder entsprechender Hilfsstoffe in Flüssigphase enthalten mittels Neutralisation und Abtrennung der gebildeten Salze, dadurch gekennzeichnet, daß man zur Gewinnung von Reinprodukten, die auch ohne Destillation niedrige Rest-Säurezahlen mit niedrigen Farbzahlen verbinden, die Neutralisation mit festen feinpulvrigen Oxiden, Carbonaten und/oder Hydroxiden der Alkali- und/oder Erdalkalimetalle bei Temperaturen oberhalb 50°C als Trockenneutralisation durchführt, dabei wenigstens zeitweise unter verringertem Druck und Abziehen auch des Neutralisationswassers arbeitet und nachfolgend die organische Flüssigphase von der feinpulvrigen Feststoffphase abtrennt.

2. Verfahren nach Anspruch 1, dadurch gekennzeichnet, daß man die Neutralisation mit den Oxiden und/oder Hydroxiden von Calcium und/oder Magnesium durchführt, wobei die wenigstens anteilsweise Mitverwendung entsprechender Calciumverbindungen bevorzugt ist.

3. Verfahren nach Ansprüchen 1 und 2, dadurch gekennzeichnet, daß man mit einer Behandlungsdauer der Neutralisation unterhalb 1 Stunde, vorzugsweise von höchstens 45 Minuten arbeitet.

4. Verfahren nach Ansprüchen 1 bis 3, dadurch gekennzeichnet, daß man die Neutralisation im Temperaturbereich von etwa 60 bis 100 °C, insbesondere im Bereich von etwa 70 bis 90 °C durchführt, wobei eine Zeitspanne für die Neutralisation von etwa 5 bis 60 Minuten besonders bevorzugt sein kann.

5. Verfahren nach Ansprüchen 1 bis 4, dadurch gekennzeichnet, daß im Druckbereich von etwa 1 bis 150 mbar gearbeitet wird.

6. Verfahren nach Ansprüchen 1 bis 5, dadurch gekennzeichnet, daß man Rest-Säurezahlen unterhalb 1,5 mg KOH/g, vorzugsweise von höchstens etwa 1 mg KOH/g bei Farbzahlen unterhalb 2 (bestimmt nach GARDNER), vorzugsweise bei Farbzahlen im Bereich von etwa 1 einstellt.

7. Verfahren nach Ansprüchen 1 bis 6, dadurch gekennzeichnet, daß man mit wasserfreien Polymerisations- bzw. Vergelungsgefährdeten olefinisch ungesättigten Einsatzmaterialien arbeitet, die auch in Abmischung mit Lösungs- und/oder Verdünnungsmitteln vorliegen können und saure Verunreinigungen (Restsäureanteile aus der Kondensation, saure Katalysatoren) enthalten.

8. Verfahren nach Ansprüchen 1 bis 7, dadurch gekennzeichnet, daß als Einsatzmaterialien schwerflüchtige Ester aus insbesondere polyfunktionellen Alkoholen und olefinisch ungesättigten Carbonsäuren, insbesondere Acrylsäure und/oder Methacrylsäure, verwendet werden.

9. Verfahren nach Ansprüchen 1 bis 8, dadurch gekennzeichnet, daß man zur Trockenneutralisation Calciumoxid und/oder -hydroxid in Abmischung mit Hydrotalcit einsetzt.

10. Verfahren nach Ansprüchen 1 bis 9, dadurch gekennzeichnet, daß man das feinpulvrige Neutralisationsmittel in wenigstens stöchiometrischer Menge, vorzugsweise in höchstens 3-fachem Überschuß, insbesondere in Mengen von etwa dem 1,3- bis 2,5-fachen der stöchiometrischen Menge, einsetzt.

**Claims**

1. A process for the production of substantially neutral, low-volatility organic compounds containing reactive olefinic double bonds from starting materials which contain these components together with small quantities of acidic reaction constituents and/or corresponding auxiliaries in the liquid phase by neutralization and separation of the salts formed, characterized in that, to obtain pure products which combine low residual acid values with low colour values, even without distillation, the neutralization is carried out as dry neutralization at temperatures above 50 °C using solid, finely powdered oxides, carbonates and/or hydroxides of the alkali and/or alkaline earth metals, reduced pressure being at least periodically applied and the water of neutralization also being removed, and the organic liquid phase is subsequently separated from the finely powdered solid phase.

2. A process as claimed in claim 1, characterized in that the neutralization is carried out with the oxides and/or hydroxides or calcium and/or magnesium, the at least partial co-use of corresponding calcium compounds being preferred.

3. A process as claimed in claims 1 and 2, characterized in that the neutralization time is under 1 hour and is preferably at most 45 minutes.

4. A process as claimed in claims 1 to 3, characterized in that the neutralization is carried out at temperatures in the range from about 60 to 100 °C and more particularly at temperatures in the range from 70 to 90 °C, a neutralization time of around 5 to 60 minutes being particularly preferable.

5. A process as claimed in claims 1 to 4, characterized in that it is carried out at temperatures in the range from about 1 to 150 mbar.

6. A process as claimed in claims 1 to 5, characterized in that residual acid values below 1.5 mg KOH/g and preferably of at most about 1 mg KOH/g are adjusted for Gardner colour standard values below 2 and preferably of around 1.

7. A process as claimed in claims 1 to 6, characterized in that water-free polymerization- or gelation-sensitive olefinically unsaturated starting materials, which may even be mixed with solvents and/or diluents and contain acidic impurities (residual acid from the condensation step, acidic catalysts), are used.

8. A process as claimed in claims 1 to 7, characterized in that low-volatility esters of, in particular, polyhydric alcohols and olefinically unsaturated carboxylic acids, more particularly acrylic acid and/or methacrylic acid, are used as starting materials.

9. A process as claimed in claims 1 to 8, characterized in that calcium oxide and/or hydroxide mixed with hydrotalcite is/are used for the dry neutralization.

10. A process as claimed in claims 1 to 9, characterized in that the finely powdered neutralizing agent is used in an at least stoichiometric quantity and preferably in an at most 3-fold excess, more particularly in quantities of around 1.3 to 2.5 times the stoichiometric quantity.

**Revendications**

1. Procédé d'obtention à l'état pur de composés organiques essentiellement neutres difficilement volatils et qui possèdent des doubles liaisons oléfiniques réactives à base de matériaux de charge qui contiennent ces composants conjointement avec des quantités inférieures de constituants de réaction acides et/ou d'adjuvants correspondants en phase liquide, au moyen d'une neutralisation et de la séparation des sels formés, caractérisé en ce que l'on effectue pour la production de produits purs qui associent également sans distillation, des indices d'acidité résiduels avec des indices de coloration bas, la neutralisation avec des oxydes des carbonates et/ou des hydroxydes solides, finement pulvérulents, de métaux alcalins et/ou alcalino-terreux à des températures au dessus de 50°C en tant que neutralisation à sec, que l'on opère pour cela au moins temporairement sous pression réduite et évacuation aussi de l'eau de neutralisation et que l'on sépare ensuite la phase liquide organique de la phase de solide finement pulvérulente.

2. Procédé selon la revendication 1, caractérisé en ce que l'on effectue la neutralisation avec les oxydes et/ou hydroxydes de calcium et/ou de magnésium, parmi lesquels l'utilisation conjointe au moins partiellement des composés du calcium correspondants est préférée.

3. Procédé selon les revendications 1 et 2, caractérisé en ce que l'on opère avec une durée de traitement de la neutralisation en dessous d'une heure, de préférence au maximum 45 minutes.

4. Procédé selon les revendications 1 à 3, caractérisé en ce que l'on effectue la neutralisation dans la plage de températures d'environ 60°C à 100°C, en particulier dans la plage d'environ 70 à 90°C, un laps de temps pour la neutralisation d'environ 5 à 60 minutes pouvant être particulièrement préféré.

5. Procédé selon les revendications 1 à 4, caractérisé en ce que l'on opère dans une zone de pressions d'environ 1 à 150 mbar.

6. Procédé selon les revendications 1 à 5, caractérisé en ce que l'on ajuste les indices d'acidité résiduels en dessous de 1,5 mg KOH/g, de préférence au maximum à environ 1 mg KOH/g pour des indices de coloration en dessous de 2 (déterminé selon Gardner), de préférence pour des indices de coloration dans la zone d'environ 1.

7. Procédé selon les revendications 1 à 6, caractérisé en ce que l'on opère avec des matériaux de charge dépourvus d'eau non saturés oléfiniquement, menacés de polymérisation ou de gélification, , qui peuvent se présenter aussi en mélange avec des solvants et/ou des agents de dilution et contenir des impuretés acides (fractions résiduelles d'acide provenant de la condensation, catalyseurs acides).

8. Procédé selon les revendications 1 à 7, caractérisé en ce que comme matériau de charge on utilise des esters difficilement volatils à partir d'alcools multifonctionnels et d'acides carboxyliques non saturés oléfiniquement, en particulier d'acide acrylique et/ou méthacrylique.

9. Procédé selon les revendications 1 à 8, caractérisé en ce que l'on met en oeuvre pour la neutralisation à sec, de l'oxyde de calcium et/ou de l'hydroxyde de calcium en mélange avec de l'hydrotalcite.

10. Procédé selon les revendications 1 à 9, caractérisé en ce que l'on met en oeuvre l'agent de neutralisation finement pulvérulent en quantité au moins stoechiométrique, de préférence en excès au maximum trois fois, en particulier en quantités allant d'environ 1,3 fois à 2,5 fois la quantité stoechiométrique.